(19) Europäisches Patentamt
European Patent Office
Office européen des brevets

(11) Publication number: 0 439 320 A1

# (12) EUROPEAN PATENT APPLICATION

(21) Application number: 91300460.2

(51) Int. Cl.$^5$: A61K 31/18

(22) Date of filing: 22.01.91

(30) Priority: 22.01.90 JP 12169/90

(43) Date of publication of application: 31.07.91 Bulletin 91/31

(84) Designated Contracting States: AT BE CH DE DK ES FR GB GR IT LI LU NL SE

(71) Applicant: YAMANOUCHI PHARMACEUTICAL CO. LTD.
No. 3-11 Nihonbashi-Honcho, 2-chome
Chuo-ku
Tokyo (JP)

(72) Inventor: Wagner, Gorm
Panum Institute, University of Copenhagen
Blegdamsvej 3C, DK-2200 Copenhagen (DK)

(74) Representative: Geering, Keith Edwin et al
REDDIE & GROSE 16 Theobalds Road
London WC1X 8PL (GB)

## (54) Erectile dysfunction treatment.

(57) The use of at least one compound selected from 5-[2-[2-(2-ethoxyphenoxy)-ethylamino]-2-methylethyl]-2-methoxybenzenesulfonamide and salts thereof for the preparation of a medicament for the prevention or treatment of erectile dysfunction.



Jouve, 18, rue Saint-Denis, 75001 PARIS

# ERECTILE DYSFUNCTION TREATMENT

This invention relates to treatment of erectile dysfunction.

Man does not have an extratunical retractor smooth muscle of the penis as do many other mammals. The cavernous smooth muscle inside the tunical albuginea acts as a retractor and by contraction keeps the penis flaccid, preventing blood pooling in the cavernous spaces. During sexual stimulation the cavernous spaces fill with blood, and the smooth muscle relaxes and is stretched ; and, at the same time the drainage from the corpus cavernosum is reduced. [cf. G.Wagner, T. Gerstenberg, & R.J. Levin, The Journal of Urology, Vol. 142, September, 723-725 (1989)]

Thus, the trabecular smooth muscle system, which is formed as a network attached to the inside of the tunica albuginea, has an important functional role, when contracted, to keep the penis flaccid but allow for erection when relaxed. Its regulation is believed to be under neural control, that is contraction by adrenergic tone and relaxation by decreased adrenergic tone with or without concomitant release of relaxing neurotransmitters.

Impotency arises from various causes, and treatment has been tried with medicaments such as a smooth muscle relaxing agent which directly relaxes the cavernous smooth muscle cells, an agent which decreases adrenergic tone, and an adrenergic nervous system blocking agent.

Known agents for increasing or strengthening erection are a lipid fraction (obtained from Cowper's gland) which contracts mouse colon, and a formulation containing a vasodilator and Azone (trade name, made by Nelson Co.) (cf.unexamined Japanese Patent Application laid open under the laying-open No.JP-A-62-175427).

However, the above known agents have various problems such as weak effect or many side effects, and so cannot be said to be satisfactory for treating impotency ; there has thus been a demand for an improved treatment agent.

The compound which is used as an active ingredient for the present invention, namely 5-[2-[2-(2-ethoxyphenoxy)ethylamino]-2-methylethyl] -2-methoxybenzene sulfonamide is represented by the following formula (I) :

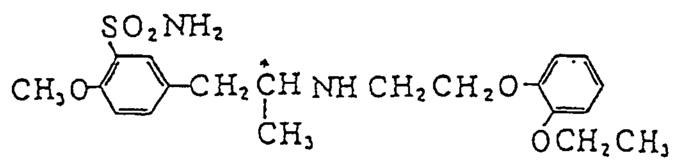

(I)

The R-(-)-isomer of compound (I) has the generic name amsulosin ; the generic name of the HCl salt of this R-(-)-isomer is amsulosin hydrochloride.

Compound (I) and its salts including amsulosin hydrochoride are known compounds first synthesized by Yamanouchi Pharmaceutical Co.Ltd. It is known that compound (I) is useful for treating hypertension, as it has a strong alpha-adrenergic receptor blocking action (examined Japanese patent publication JP-A-62-52742). It is also known that compound (I) and its salts can be used for treating cardiac insufficiency and lower urinary tract dysfunction (unexamined Japanese patent Application laid open under the laying open No.JP-A-62-9).

However, it has not been known that compound (I) and its salts are useful for treating erectile dysfunction. These compounds in fact have strong activity for this purpose, and low toxicity.

Compound (I) can readily form salts, including, for example, acid addition salts with inorganic acids such as mineral acids (e.g. hydrochloric, sulfuric, nitric, phosphoric and hydrobromic acids and the like) and with various organic acids such as acetic, oxalic, malonic, succinic, maleic, fumaric, lactic, malic, tartaric, salicyclic, gallic, picric, methanesulfonic and ethanesulfonic acids and the like ; acidic amino acids such as glutamic and aspartic acids also form such salts useful in the invention.

Compound (I) and its salts are readily made as shown in the before-mentioned unexamined Japanese patent publication No. JP-A-62-52742.

"Erectile dysfunction" in this invention includes "impotence", "impotency", "asynodia", "invirility", etc. and the present invention is applicable to all such dysfunctions. "Erectile dysfunction" in the present invention is not limited to specific symptoms, but covers all of functional, psychic and organic dysfunctions.

The effectiveness of compound (I) and its salts for treating impotence is shown by the following pharmacological tests.

(1) Relaxation of cavernous smooth muscle :

The relaxing effect of compound (I) on isolated specimens of penis cavernous smooth muscle of pigs

(smooth muscle strips obtained from slaughtered boards) and of humans was tested by the following method.

**Test Method**

The pig and humans cavernous smooth muscle specimens are placed in an organ bath, and the tension is recorded.

In the case of isolated strip of pig cavernous smooth muscle, the strip was precontracted with a noradrenaline dose of $10^{-6}$ to $10^{-8}$ M, and the strip was then treated with compound (I) ($10^{-8}$ M) ; five minutes after the treament with compound (I), the strip was treated with the same dose of noradrenaline as before, with further repetition of this noradrenaline dosage at intervals of 35 to 40 minutes. The recovery from noradrenaline contraction and the relaxatory effect of compound (I) were evaluated. Work was also conducted with reference compounds in the porcine tissue, measuring the contractile response to noradrenaline.

In the case of human cavernous smooth muscle strip, the relaxatory effect was evaluated. The specimen was contracted with a noradrenaline dose of $2 \times 10^{-5}$ M and was treated with compound (I) at a dose of $10^{-7}$ M.

**Results**

Table 1 shows the antagonist dose and the time until full contractile response was restored in the porcine tests.

TABLE 1

| Test Compound (Antagonist) | Dose (M) | Time until prior response restored (mins) |
|---|---|---|
| Compound (I) | $10^{-8}$ | At 165 min 60% of full response seen |
| Prazocin | $10^{-6}$ | 160 |
| Phentolamine | $7 \times 10^{-7}$ | 150 |
| Papaverine | $2 \times 10^{-4}$ | 45 |
| VIP (vasoactive intestinal peptide) | $10^{-7}$ | 45 |

In Table 2, the result for human tissue is shown.

TABLE 2

| Test Compound | Dose (M) | Inhibiting effect (%) |
|---|---|---|
| Compound (I) | $10^{-7}$ | 100 |

(2) Effects on penis erection (tumescence) and its firmness (rigidity)

Tumescence and rigidity after intracorporeal injection of compound (I) were evaluated for monkeys.

**Test Method**

Anesthetized monkeys (Cynogos or Vervet), 4-6 kg body weight, were used for the test.

The animal was anesthetized with ketamine and 0.1 g of compound (I) was injected in solution into the corpus cavernosum with a tourniquet at the base of the penis ; the animal was then under observation and changes in tumescence and rigidity were noted and plotted. The tourniquet was removed 5 min after the injection.

Manual and visual evaluation of tumescence and rigidity was made 5, 10, 15, 20, 30, 60 and 120 min after injection.

**Results**

The activity of compound (I) (dose : 0.1 g) on penis erection (tumescence) and firmness (rigidity) in the anesthetized monkeys is shown in Fig. 1.

(3) Acute Toxicity Test

The acute toxicity of compound (I) salt was evaluated using ICR mice and F344 rats.

**Test Method**

compound (I) was dissolved in dimethylsulfoxide, and the same amount of Macrogol 400 (trade name) was added, and then distilled water for injection was added thereto to provide a solution of predetermined concentration. The solution was injected once each test, intravenously or subcutaneously, at a rate of 10-20 ml/kg, the maximum concentration of the administered solution being 12 mg/ml and 25 mg/ml respectively. The animals were kept under observation for 2 weeks. $LD_{50}$ values were determined by the up and down method (Probit method) using 5 animals.

TABLE 3 : $LD_{50}$ in mice and rats in the case of intravenous and subcutaneous administration (mg/kg)

| Animals | Administration Route | $LD_{50}$ (95% confidence limits) Male | Female |
|---|---|---|---|
| FCR mice | intravenous | 98 ( 88-108) | 103 ( 91-111) |
| | subcutaneous | 254 (224-289) | 275 (237-341) |
| F344 rats | intravenous | 70 ( 60- 80) | 78 ( 68- 91) |
| | subcutaneous | 395 (356-452) | 347 (300-407) |

As apparent from the above results, compound (I) and its salts have strong relaxing activity on cavernous smooth muscle and have excellent effects of potent penis erection, tumescence and rigidity with long lasting action and with low toxicity.

Thus, compound (I) and its salts can be used clinically for treatment of erectile dysfunction.

For clinical use, the compound or salt is used in medical or pharmaceutical compositions with carriers or diluents which can be used generally for preparing medicaments. The compound or salt may be administered orally or parenterally for the purpose of general administration.

That is, as admisnistration

routes of the present compositions, there may be oral administration in the form of tablets, pills, granules, powders or capsules, and parenteral administration in the form of injection or patches. But it is preferred for clinical purposes to use compositions for local administration ; that is, it is preferred to use external preparations (e.g. liquid preparations, ointments, gel ointments, cream preparations, spray preparations, emulsions, lotions, patch preparations such as cataplasm, plaster) or injections for local administration. The dosage should be determined with consideration given to administration route, dysfunction condition and other factors of particular patients, but is normally 0.01 to 50 g in the case of injections for local administration, and 4 to 20 g in the case of external preparations for local administration such as spray preparations and gel ointments ; it is possible to administer the medicament in a single dose or in several divided doses. According to the form of pharmaceutical preparation, it may be sprayed or painted several times to reach the required total dose.

Compound (I) and its salts have markedly low percutaneous absorption. Thus, to achieve good clinical effect at low dosage it is preferred to use preparations having increased percutaneous absorption. For example, there may be used a method like that disclosed in EP patent application No. 90309439.9 (EP-A- ), with the active ingredient being used in a preparation containing at least one transdermal absorption promoter selected from aliphatic dicarboxylic acid diesters, nicotinic acid esters and isonicotinic acid esters, with or without a terpene, usually is the presence of aqueous alcohol.

The present invention is further explained by the following fomulation examples.

**Formulation Example 1 (Injection)**

2 mg of amsulosin hydrochloride and 9 g of sodium chloride were dissolved in 1 litre of distilled water for injection with warning.

After filtering the solution through a bacterial filter, a predetermined volume of the solution was poured into a glass ampoule under sterile conditions to provide an injection for treatment of erectile dysfunction.

**Formulation Example 2 (Spray)**

2.0 g of amsulosin hydrochloride was dissolved in a mixture of 46.5 g of ethanol and 46.5 g of Michaelis buffer (pH : 7) with warming. After adding to the solution 5.0 g of diethyl sebacate (penetration accelerating agent), the mixture was thoroughly stirred to provide a uniform solution. 10 ml of the solution was charged into a pressure-type spray vessel (contents : 15 ml ; spraying volume for single dose : 0. 05 ml) to provide a spray formulation for treatment of erectile dysfunction.

**Formulation Example 3 (Spray)**

2.0 g of amsulosin hydrochloride was dissolved in a mixture of 44.0g of ethanol, 5.0g of propylene glycol, and 44.0 g of Michaelis buffer (pH : 7) with warming.

After adding to the solution 5.0 g of diethyl sebacate, the mixture was well stirred to form a uniform solution.

In similar manner to the above Formulation Example 2, a spray formulation for treatment or erectile dysfunction was obtained.

**Formulation Example 4 (Gel Ointment)**

1.0 g of Carbopol 940 (trade name) was swelled in 30 g of purified water with stirring, and 0.5 g of diisopropanolamine was added thereto to form a gel. 3.0 g of amsulosin hydrochloride was dissolved in a mixture of 46 g of ethanol and 46 g of Michaelis buffer (pH : 7), and after adding thereto 5.0 g of diethyl sebacate, the mixture was well stirred to form a uniform solution. 68.5 g of the solution thus obtained was added into the Carbopol gel obtained above, and the mixture was well kneaded to give a gel ointment for treatment of erectile dysfunction.

**4. Explanation of the drawings**

Fig.1 shows the effect of amsulosin hydrochloride on penis erection (tumescence) and firmness (rigidity).

## Claims

1. The use of at least one compound selected from 5-[2-[2-(2-ethoxyphenoxy)ethylamino]-2-methylethyl]-2-methoxybenzenesulfonamide and salts thereof for the preparation of a medicament for the prevention or treatment of erectile dysfunction.

2. A composition for treating erectile dysfunction which comprises at least one compound selected from 5-[2-[2-(2-ethoxyphenoxy)-ethylamino]-2-methylethyl]-2-methoxybenzenesulfonamide and its salts.

Fig. 1

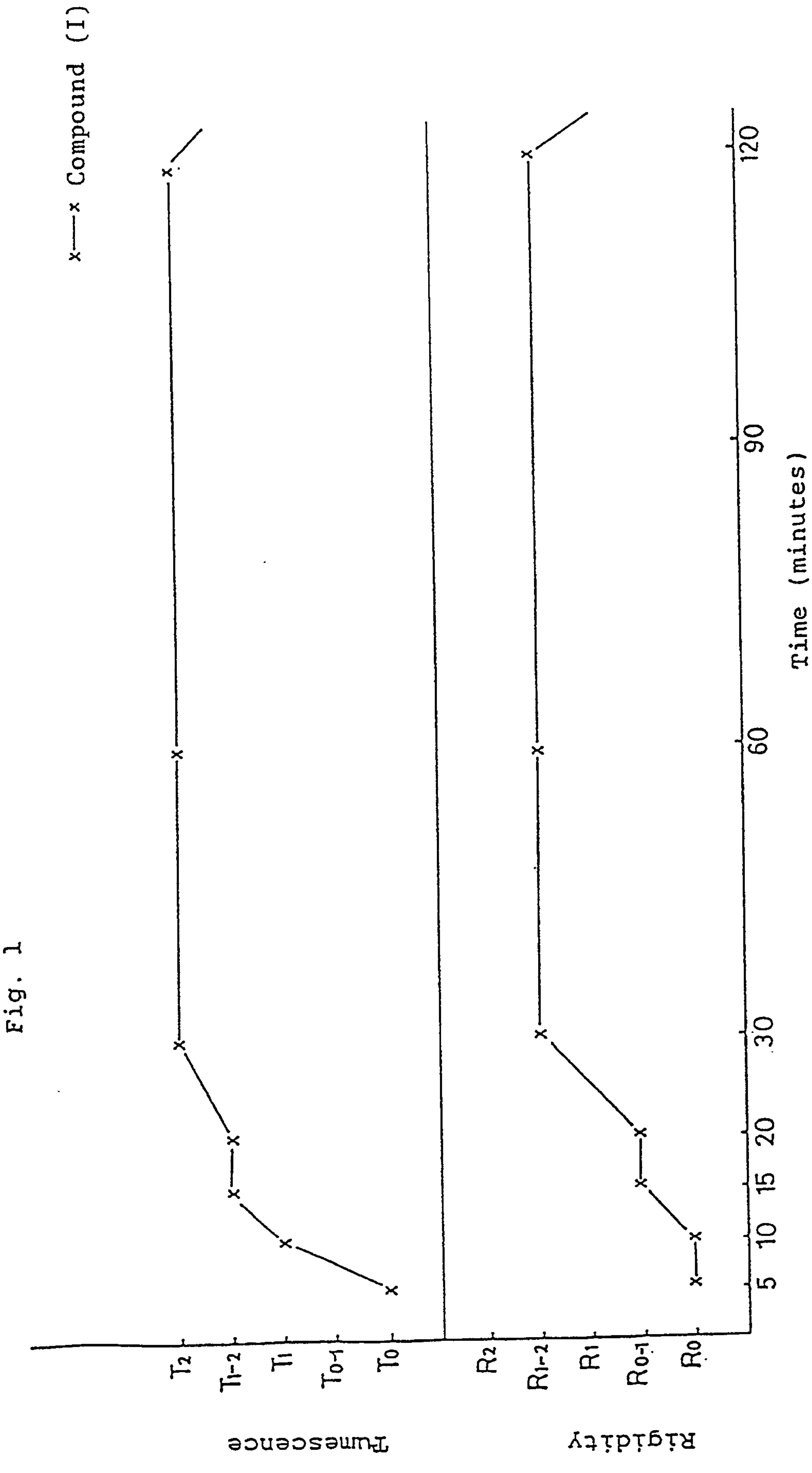
x——x Compound (I)
Tumescence
T2
T1-2
T1
T0-1
T0
Rigidity
R2
R1-2
R1
R0-1
R0
5
10
15
20
30
60
90
120
Time (minutes)

European Patent Office

# EUROPEAN SEARCH REPORT

Application Number

EP 91 30 0460

| Category | DOCUMENTS CONSIDERED TO BE RELEVANT<br>Citation of document with indication, where appropriate, of relevant passages | Relevant to claim | CLASSIFICATION OF THE APPLICATION (Int. Cl.5) |
|---|---|---|---|
| X,P | EUROPEAN JOURNAL OF PHARMACOLOGY, vol. 186, no. 1, 1990, pages 87-93, Elsevier Science Publishers B.V. (Biomedical Division); F. HOLMQUIST et al.: "Effects of the alpha1-adrenoceptor antagonist R-(-)-YM12617 on isolated human penile erectile tissue vas deferens"<br>* Page 92 * | 1 | A 61 K 31/18 |
| Y | NAUNYN-SCHMIED. ARCH. PHARMACOL., vol. 330, no. 1, 1985, pages 16-21, Springer-Verlag; HONDA et al.: "Alpha1-adrenoceptor subtype mediating contraction of the smooth muscle in the lower urinary tract and prostate of rabbits"<br>* Page 16, right-hand column, lines 34-38; page 19, left-hand column, lines 28-31; page 21, left-hand column, lines 17-22 * | 1 | |
| | | | TECHNICAL FIELDS SEARCHED (Int. Cl.5) |
| Y | J. AUTOM. PHARMAC., vol. 5, 1985, pages 81-88; H. HEDLUND et al.: "Comparison of the responses to drugs acting on adrenoreceptors and muscarinic receptors in human isolated corpus cavernosum and cavernous artery"<br>* Page 81, left-hand column; page 86, right-hand column, line 18 - page 87, left-hand column, line 50; page 88, left-hand column, lines 15-18 * | 1 | A 61 K |
| X,D | EP-A-0 194 838 (YAMANOUCHI)<br>* Column 3, lines 55-65 * & JP-A-62-9<br>-/- | 2 | |

The present search report has been drawn up for all claims

| Place of search | Date of completion of the search | Examiner |
|---|---|---|
| THE HAGUE | 09-04-1991 | GERLI P.F.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)

Page 2

European Patent Office

# EUROPEAN SEARCH REPORT

Application Number

EP 91 30 0460

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | J. AUTONOM. PHARMACOL., vol. 5, 1985, pages 261-270; H. HEDLUND et al.: "Effect of drugs interacting with adrenoreceptors and muscarinic receptors in the epididymal and prostatic parts of the human isolated vas deferens" * Page 265, left-hand column, line 53 - page 266, left-hand column, line 19; page 268, right-hand column, lines 2-17; page 269, right-hand column, lines 15-18 * ----- | 1 | |
| | | | TECHNICAL FIELDS SEARCHED (Int. Cl.5) |

The present search report has been drawn up for all claims

| Place of search | Date of completion of the search | Examiner |
|---|---|---|
| THE HAGUE | 09-04-1991 | GERLI P.F.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)